# EUROPEAN PATENT SPECIFICATION

(11) **EP 1 249 232 B1**
(45) Date of publication and mention of the grant of the patent: **11.10.2006**
(21) Application number: 01113963.1
(22) Date of filing: 08.06.2001
(51) Int. Cl.: A61K 9/51

(54) **Controlled release nanoparticles for percutaneous use and composition containing the same**
Nanopartikel mit verzögerter Freisetzung zur perkutanen Anwendung sowie diese enthaltende Zusammensetzung
Nanoparticles à libération contrôlée pour application percutanée et composition les contenant

(30) Priority: 13.04.2001 KR 2001019869
(43) Date of publication of application: 16.10.2002
(73) Proprietor: PACIFIC CORPORATION, Seoul 140-777 (KR)
(72) Inventor: Kwon, Sun Sang, Yongin-city, Kyungki-do (KR); Yoo, Byung Hee, No. 401-201, Paldal-ku, Suwon-city, Kyungki-do (KR); Nam, Yoon Sung, No. 901-404, Jookong APT, Suji-up, Yongin-city, Kyungki-do (KR); Kim, Bae Hwan, Paldal-ku, Suwon-city, Kyungki-do (KR); Ku, Bong Seok, Seocho-ku, Seoul (KR); Han, Sang Hoon, Suwon-city, Kyungki-do (KR); Jang, Li Sub, Suji-up, Yongin-city, Kyungki-do (KR); Lee, Jong Suk, Yongin-city, Kyungki-do (KR)
(74) Representative: Maisch, Thomas

(56) References cited:
- EP-A- 0 447 318
- DE-A- 19 619 150

## Description

### BACKGROUND OF THE INVENTION

### 1. FIELD OF THE INVENTION

The present invention relates to a percutaneous controlled releasing material using nano-sized polymer particles and an external application agent containing the material. More specifically, the present invention provides a percutaneous agent which uses a 2-step of bio-effective material transferring mechanism comprising; making particles with a few to a few hundred nanometers of diameter by using bio-compatible polymers, attaching a physiologically active agent into the bio-compatible polymer and applying the bio-compatible polymer particles with bio-active agent on the skin, wherein diameters of the bio-compatible polymer particles are controlled to penetrate into the skin through the stratum corneum but are not so small to penetrate into dermis (step 1); and slowly effusing the bio-active agent into the skin while the bio-compatible polymer particles remain on the dermis(step 2). The present invention also provides an external application agent using the bio-compatible polymer particles.

### 2. DESCRIPTION OF THE PRIOR ART

The skin, as the primary protect shield of the human body, shields the internal organs from the potentially damaging stimuli such as circumstance changes, ultra violet rays, pollutants, etc. Recently, a lot of efforts to suppress aging of the skin and to maintain healthy and beautiful skin have been undertaken. For example, as an effort to maintain skin function and to suppress the aging and melanin accumulation of the skin, physiologically active materials obtained from animals, plants and microorganisms have been used as components of cosmetic compositions.

Especially, percutaneous methods of absorbing effective components directly through the skin have been much studied. Such percutaneous absorbing method is described below.

As a basic method, the physiologically active material is transferred into the skin by dissolving the active material in a suitable solvent and applying the solution to the skin. In this case, there are some bottlenecks in selecting solvents complying with the active materials. However, because such solvents cause irritation and it is hard to control the tactile sensation, above method has many difficulties preventing its commercial use, and has recently nearly disappeared.

Following the above dissolving method, emulsion-type percutaneous releasing agent to improve skin absorption has been developed. The technology has developed from the early method of containing the active agent into micrometer-sized emulsion particles to a method of containing the active agent into the nanometer-sized emulsion particles. Specifically, a technology for making nanometer to micrometer-sized emulsion particles using effective agents, lipids, glycerol, water, phospholipid or water-soluble non-ionic sufactants is disclosed in USP 5,338,761. Preparing nano-sized particles using charged-lipid as an emulsifier is disclosed in USP 6,120,751. Further, a method for preparing nano-sized particles using micro-emulsion, obtained when three phases consisting of emulsifier, oil and water become balances, is disclosed in USP 5,152,923, WO 91/06,286 and WO 91/06,287.

EP-A-0 447 318 discloses polymer nanoparticles comprising a physiologically active agent suitable for the percutaneous delivery of the active agent. The size of the particles is for example 195 nm.

However, when an unstable active agent is deposited in the emulsion particle, because the emulsion membrane kinetically equilibrates with the outer phase, the active agent continuously contacts the water, which causes oxidation and dissolution of the active agents. Further, the membrane of the emulsion is physically very weak and chemically unstable, so that the membrane of the emulsion is easily broken by organic or inorganic pollutants. Also, because the emulsion is very sensitive to the light, it is very hard to store the emulsion for a long time. As seen above, the nano-sized emulsion that is made by using small molecular emulsifying agent is not suitable for unstable active agent, and there are many obstacles to make commercial goods. Further, a lot of emulsifiers are needed to contain a sufficient amount of active agent, which may cause skin irritation.

To overcome the above problems, methods for stabilizing the active agent against light, moisture and oxygen of air and for developing sustained releasing characteristics of the the active agent have been studied. As a result, capsule-type formulation and patch-type formulation are introduced.

As a sustained releasing formulation, there is a patch formulation wherein aqueous or oily active agent is suspended in the gel and percutaneous absorption is performed by applying the gel onto the skin for a long time. In this patch formulation, a method of covering the patch with shield was developed, wherein the inner side of the polymer matrix contacts the skin and the outer side of the polymer matrix blocks outer air and light, when suspending active agent into the polymer matrix. However, there are some faults with this method; it takes much time for the active agent to be absorbed into the skin, and therefore, it needs binding agents to make the polymer matrix to stay on the skin for a long time.

There is another method of containing the active agent in a hard capsule, specifically, using small particles such as a micrometer-sized particle. As an example, a sustained releasing formulation which release the agent slowly while having the micro capsule stay on the skin for a long time is disclosed in USP 5,286,495, WO 89/08,449 and WO 88/01.213. In case of the micro capsule formulation, stabilization is acquired by capturing the active agent in the capsule. However, because the absorption rate of the micro capsule into the skin is not so high, the percutaneous formulation using the capsule should stay on the skin for a long time, and therefore the formulation is expected to contact the air, light and moisture, so that the active agent within the capsule is inactivated or metamorphosed, and skin irritation increases. Further, absorption rate of the active agent is not so high and the micro- sized particle is uncomfortable to the touch.

As seen above, conventional topical absorption methods have some problems such as low absorption rate, irritation of the skin and difficult stabilization of the active agent. Therefore, a new percutaneous sustained-releasing formulation superior to the conventional formulation is needed.

### SUMMARY OF THE INVENTION

To solve the above problems, applicants of the present invention studied percutaneous releasing material having such characteristics as 1) a highly stabile active agent in the formulation, 2) high topical absorption rate, 3) decreased irritation of the skin and improved tactile comfort, and 4) applicability to various active agents.

As a result, the applicants found that above the problems can be solved by using nanometer-sized polymer particles.

Thus, an object of the present invention is to provide a percutaneous releasing material having such characteristics as high stability of the active agent in the formulation, high topical absorption rate, decreased irritation of the skin, increased tactile comfort and applicability to various active agents.

Further, another object of the present invention is to provide an external application agent composition.

To accomplish the above objects, the present invention provides a percutaneous releasing material, an external application agent and a method for preparing the percutaneous releasing material according to claims 1,5 and 8.

Polymer particles having diameters of 30 to 150nm and more preferably the diameter is 50nm.

In the present invention, "nano-particle" is referred to as a particle having a diameter of nanometer scale.

The physiologically active agent used in the present invention preferably comprises medicaments such as antibiotics, antitumor agent, anti-inflammatory agent, antipyretic, analgesia, anti-edema agent, antitussive agent, expectorant, depressant, muscle relaxant, antiepileptic, anti-ulcer agent, anti-melancholia agent, anti-allergy agent, cardiotonic agent, anti-arrhythmic agent, vasodilatin, hypotensive agent, antidiabetic, homoeostasis agent, polypeptide, hormone, antioxidant, hair growing agent, hair tonic, gumboil agent (antimicrobial agent), whitening agent, crease resistant or disapproval agent such as collagen synthesizing accelerant, membrane fortifier and moisturizing agent.

The polymers used in the present invention are natural or synthetic polymers, which are biocompatible, and may be used individually, in combination with each other or in bridged composition. Further, biodegradable or non-biodegradable polymers may be used together.

In the present invention, the percutaneous agent is in state that the nanometer- sized particles are suspended in the solution. The content of the nano-particle in the aqueous solution is 0.0001 percent by weight to 90 percent by weight, preferably 0.1 percent by weight to 50 percent by weight, and more preferably 5 percent by weight.

The composition of the external applying of the present invention is not restricted in formulation. For example, the formulation may be cosmetics such as skin freshener, moisturizing preparation, massage cream, nutrient cream, pack, gel, skin-adhesive cosmetic, lipstick, make-up base, foundation, etc.; washing compositions such as shampoo, rinse, body-cleanser, soap, toothpaste, mouth wash, etc.; or percutaneous medicament formulation such as lotion, ointment, gel, cream, patch, spray, formulations for hair growth, etc.

These and other features of the present invention will be well understood by one skilled in the art from the following detailed descriptions.

### BRIEF DESCRIPTION OF THE DRAWINGS

Fig. 1 is the diameter distribution of the nano particles prepared in examples 1 to 3.
Fig. 2 shows the distribution of the particles prepared in example 4 observed by transmission electron microscopy.
Fig. 3 shows the distribution of the particles prepared in example 5 observed by transmission electron microscopy.
Fig. 4 shows the distribution of the particles prepared in example 6 observed by transmission electron microscopy.
Fig. 5 shows fluorescent PMMA polymers with diameters of about 50nm being absorbed into the skin.
Fig. 6 shows fluorescent PMMA polymers with diameters of about 80nm being absorbed into the skin.

The present invention will be described in detail hereinafter.

The present invention provides a transferring mechanism which comprises making particles by using bio-compatible polymers, then attaching a physiologically active agent into the polymer and applying the polymers infused with physiologically active agent to the skin. The diameters of the bio-compatible polymer particles are controlled in order to penetrate into the skin across the stratum corneum but not to penetrate into dermis. The diameters of the particles are within 30 to 150 nm.

After penetrating into the skin across the stratum corneum but not into dermis (step 1), the physiologically active agents are slowly effused into the skin while the bio-compatible polymer particles remain in the upper layer of the dermis (step 2). The polymeric nano-particles penetrate into the middle of the skin, i.e., exterior to the dermis, and effuse the physiological active agent contained in the particle. The active agent effused improves the activation of the skin cells, whitening and smoothing the wrinkles, providing anti-oxidization and moisturizing effects, and therefore enhancing skin's ability to protect.

The nano-particles, which effused the active agent while staying in the middle of the skin, go out of the skin in accordance with the turn-over period of the skin, either being separated and removed from the skin or decomposed by enzymes. Therefore, side effects caused by the accumulation of the nano particles can be avoided.

That is, the present invention provides 2-step of a percutaneous absorption method comprising a percutaneous absorption step in which the nano particles containing active agent is transferred into the exterior upper layer of the dermis and diffused (step 1); and a sustained releasing step in which active agents are slowly released into the dermis from the nano particles (step 2).

To accomplish the above mechanism, after preparing various-sized nano-particles, the size which can transfer the horny layer of the skin, contain active agent with a stable state, and transfer into the upper layer of the dermis when administrated on the skin, then release the active agent contained therein while staying in the upper layer of the dermis is determined. Further, because unexpected side effects may occur when the particles are accumulated in the skin after releasing the active agent, the size is determined considering their ability to be removed from the skin in accordance with the turn-over period of the skin and, further, that they can be decomposed by the enzymes to small organic compounds.

Hundreds of trials show that particles having diameters of 1 to 500 nm can accomplish such conditions. When the particle is smaller than 1 nm, although transfer is excellent, the particle is transferred so well that it is hard to remove the particles and containing capacity is not sufficient. On the other hand, when the particle is bigger than 500 nm, containing capacity is excellent but transfer is not sufficient.

Any carrier conventionally used in the percutaneous absorption may be used in the present invention, on the condition that the particle size is restricted as shown above.

There is no restriction in the polymer used in the present invention. Any of the below polymers, for example, may be used in the present invention: natural polymers such as acacia gum, Irish moss, karaya gum, gum tragacanth, gum guaiac, xanthan gum, locust bean gum and derivatives thereof; proteins such as casein, gelatin, collagen, albumin, globulin, fibrin and derivatives thereof; natural carbohydrates such as cellulose, dextrin, pectin, starch, agar, mannan and derivatives thereof; polyvinyl polymers and derivatives thereof (ex: polyvinylpyrrolidon, polyvinyl alcohol, polyvinylmethylether, polyvinylether, etc.); polycarboxylic acids and derivatives thereof (ex: polyacrylic acid, polymetacrylic acid, polymethylmetacrylate, etc.); hydrocarbons such as polyethylene, polypropylene and isomers thereof; and polysaccharide and its derivatives (ex: polysucrose, polyglucose, polylactose and salts thereof). Further, bridged types of the above polymers may be used too. Natural polymers are known as good physiologic material, and above polymers also have good physiologic properties, therefore, they can be used for preparing nano particles of the present invention.

In addition, other polymers, for example, fatty acid polymers (ex: polylactic acid, polyglycole acid, polymalin acid) and derivatives thereof; poly-, α-, cynoacrylic acids and derivatives thereof; poly- β-, hydroxybutyric acid, polyalkylene oxalate (ex: polymethylene oxalate, polytetramethylene oxalate, etc), polyorthoesters, polyorthocarbonates, polycarbonates (ex: polyethylene carbpnate, polyethylene- propylene carbonate, etc.) and polyamino acid (ex: poly-γ-benzylglutamic acid, polyalanine, poly-γ-methylglutamic acid, etc) may be used. Further, polymers which are excellent in physiologic fitness but poor in biodecomposition character such as polystyrene, polyacrylic acid and its derivatives, polymetacrylic acid and its derivatives, acrylate-metacrylate copolymer, polyamide (ex: nylon), polyethyleneterephthalate, polyamino acid, silicon polymers, dextranstearate, ethylcellulose, acetylcellulose, nitrocellulose, polyurethane, dehydrated maleate copolymer, ethylene-vinylacetate copolymer, polyvinylacetate, polyvinyl alcohol and polyacrylamide may be used individually, and copolymers and mixtures thereof may be used. Derivatives and salts thereof also may be used.

In the above polymers, polymethylmetacrylate is excellent, because it is good in both physiological fitness and releasing the active agent into the dermis while staying in the upper layer of the dermis without decomposition. Polymers, which decompose in vivo, are excellent in releasing the active agent into the dermis while staying in the upper layer of the dermis.

There is no restriction in the physiologically active agent contained in the nano-particle, for example, antibiotics such as gentamicin, dibekacin, kanendomycin, lividomycin, tobramycin, amikacin, fradiomycin, sisomicin, tetracycline hydrochloride, oxytetracycline hydrochloride, rolitetracycline, doxycycline hydrochloride, ampicillin, piperacillin, ticarcillin, cephalothin, cephaloridine, cefotiam, cefsulodin, cefmenoxime, cefmetazole, cefazolin, cefotaxime, cefoperazone, ceftizoxime, moxolactam, latamoxef, thienamycin, sulfazecin and azthreonam; antitumor agents such as bleomycin hydrochloride, methotrexate, actinomycin D, mitomycin C, vinblastine sulfate, vincristine sulfate, daunorubicin hydrochloride, adriamycin, neocarcinostatin, cytosine arabinoside, fluorouracil, tetrahydrofuryl-5-fluorouracil, krestin, picibanil, lentinan, levamisole, bestatin, azimexon, glycyrrhizin, poly I:C, poly A:U and poly ICLC; anti-flamatory agents such as sodium salicylate, sulpyrine, sodium flufenamate, sodium diclofenac, sodium indomethacin, morphine hydrochloride, pethidine hydrochloride, levorphanol tartrate and oxymorphone; antipyretics; analgesia; anti-edema agent; expectorant and antitussive agents such as ephedrine hydrochloride, methylephedrine hydrochloride, noscapine hydrochloride, codeine phosphate, dihydrocodeine phosphate, alloclamide hydrochloride, chlophedianol hydrochloride, picoperidamine hydrochloride, cloperastine, protokylol hydrochloride, isoproterenol hydrochloride, salbutamol sulfate and terbutaline sulfate; depressant such as chlorpromazine hydrochloride, prochlorperazine, trifluoperazine, atropine sulfate and scopolamine methylbromide; muscle relaxant such as pridinol methanesulfonate, tubocurarine chloride and pancuronium bromide; antiepileptics such as sodium phenytoin, ethosuximide, sodium acetazolamide and chlordiazepoxide hydrochloride; anti-ulcer agents such as metoclopramide and L-histidine monohydrochloride; anti-melancholia agents such as imipramine, clomipramine, noxiptiline and phenelzine sulfate; anti-allergy agents such as diphenhydramine hydrochloride, chlorpheniramine maleate, tripelenamine hydrochloride, methdilazine hydrochloride, clemizole hydrochloride, diphenylpyraline hydrochloride and methoxyphenamine hydrochloride; cardiotonic agents such as trans-p-oxocamphor, theophyllol, aminophylline and etilefrine hydrochloride; anti-arrhythmetic agents such as propranolol hydrochloride, alprenolol hydrochloride, bufetolol hydrochloride and oxyprenolol hydrochloride; vasodilatins such as oxyfedrine hydrochloride, diltiazem hydrochloride, tolazoline hydrochloride, hexobendine and bamethan sulfate; hypotensive agents such as hexamethonium bromide, pentolinium, mecamlamine hydrochloride, ecarazine hydrochloride and clonidine hydrochloride; antidiabetics such as sodium glymidine, glypizide, phenformin hydrochloride, buformin hydrochloride and metformin; anti-precipitants such as sodium heparin and sodium citrate; homoeostasis agents such as thromboplastin, thrombin, menadione sodium bisulfite, acetomenaphthone, e-amino-caproic acid, tranexamic acid, carbazochrome sodium sulfonate and adrenochrome monoaminoguanidine methanesulfonate; antituberculous agents such as isoniazid, ethambutol and sodium p-aminosalicylate; insulin, somatostatin, derivative of somatostatin, growth hormones, prolactin, adrenocorticotropic hormone (ACTH), melanocyte stimulating hormone (MSH), thyroid hormone releasing hormone (TRH) and its salts and derivatives; thyroid stimulating hormone (TSH), luteinizing hormone (LH), follicle stimulating hormone (FSH), vasopressin, derivative of vasopressin, oxytocin, calcitonin, parathyroid hormone, glucagon, gastrin, secretin, pancreozymin, cholecystokinin, angiotensin, human placental lactogen, human chorionic gonadotropin (HCG), enkephalin, derivative of enkephalin, endorphin, kyotorphin, interferons (a, b, g), interleukins (I, II, and III), tuftsin, thymopoietin, thymosin, thymostimulin, thymic humoral factor (THF), serum thymic factor (FTS) and derivatives thereof; thymic factors, tumor necrosis factor (TNF), colony stimulating factor (CSF), motilin, dinorphin, bombesin, neurotensin, cerulein, bradykinin, urokinase, asparaginase kallikrein, substance P analogue and antagonist; polypeptides such as nerve growth factor, blood coagulation factors VIII, IX, lysozyme chloride, polymixin B, colistin, gramicidin, bacitracin, protein synthesis stimulating peptides, gastric inhibitory polypeptide (GIP), vasoactive intestinal polypeptide (VIP), platelet-derived growth factor (PDGF), growth hormone releasing factor (GRF, somatocrinin), bone morphogenetic protein (BMP), epidermal growth factor (EGF); hormone medicaments such as prednisolone succinate, prednisolone sodium phosphate, dexamethasone sodium sulfate, betamethasone sodium phosphate, hexestrol phosphate, hexestrol acetate and methimazole; antioxidants such as coenzyme Q10(co-Q10), vineatrol (resvaratrol), BHT, vitamin A, derivative of vitamin A, derivative of vitamin C, vitamin E and derivatives of vitamin E; antimicrobial agents such as tricolosan, chlorohexidine, cetylpyridinium chloride and natural oil; growing hair agents and hair tonic such as minoxidil, TGF (transforming growth factor), EGF (epidermal growth factor), FGF (fibroblast growth factor), IGF (insuline-like growth factor) testosterone and androgen; whitening agent; crease resistant and disapproval agent such as chollagen synthesizing accelerant; membrane fortifying and moisturizing agents such as ceramide and spingo acid; may be used as physiologically active agent. The content and the sorts of the active agents contained in the nano-particles are controlled according to the cases and the objects to be used.

The above agents are transferred by polymers suitable for each agent, according to the type of medicine and its chemical and physical characteristics. The amount of the polymer is 0.1 to 100 times the weight of the agent used, preferably 1 to 50 times the weight of the agent used.

The present invention will be embodied by way of the following examples.

### Examples

The method of preparing the nano particles containing the active agents is not restricted, and PMMA (polymethylmetacrylate) is used as a polymer to produce nano-particles in the present examples. Molecular weights between 5,000 and 1,000,000 of the PMMAs may be used, and the PMMA having molecular weight of 75,000 is used in the present examples. Active agents to be contained in the nanoparticles are not restricted, and retinol, coenzyme Q10 (hereafter, referred as co-Q10) and resveratrol are used in the present examples.

In the present examples, microfluidizer (Microfluidics Corporation, U.S.A.) is used for making emulsions for unique and small nanometer sized particles. Pressure is controlled between 500 bar and 1,500 bar and flow is controlled between 20 ml/min to 150ml/min. Surfactant is used to emulsify the oil phase and aqueous phase, and SLS (sodium laurylsulfate) is used in the present examples.

Organic solvent used to dissolve the polymer and the active agent is selected from solvents characterized as not water-soluble and not so high in their boiling points. For example, halogenated alkane (chloromethane, dichlorometnane, chloroform, tetrachloroforomethane, dichloroethane), ethylacetate, diethylether, cyclohexane, benzene, toluene and mixtures thereof may be used the solvent. The amount of the organic solvent used in the first emulsifying is 3 to 50 times the volume of the total active agent and polymer, preferably 7 to 12 times the volume.

Surfactants are used in the first emulsifying step and nano-emulsion preparation step. For example, anionic surfactant (ex: sodium oleate, sodium stearate, sodium laurylsulfate), nonionic surfactant (ex: polyoxyethylenesolbitan fatty acid esters [Tween 80, Tween 60, products of Atlas Powder Co., U.S.A.], cationic surfactant and amphoteric surfactant may be used, and ausiliary surfactants such as polyvinylpyrrolidon, polyvinyl alcohol, carboxymethylcellulose, lecithin, gelatin and low molecular alcohol may be added to the above surfactants. The content of the surfactant is 0.1 to 20 %, and preferably 0.5 to 5%.

Hereinafter, examples of preparing nano particles containing active agent are specifically described.

### Examples 1 to 9

PMMA (molecular weight 75,000) was used as a polymer, and retinol, co-Q10 and resveratrol were used as active agents. Each of 2.52g of co-Q10 as an active agent and 4.00g of PMMA as a polymer were dissolved in the 56ml of dichloromethane homogeneously to make the oil phase. Then, the above oil phase was added to the 400ml of aqueous solution in which 2g of a surfactant (SLS) was dissolved to accomplish the first emulsifying. For the first emulsifying, the above mixture was treated by the homogenizing-mixer at 5,000 rpm for 3 minutes, then proceeded into the microfluidizer to prepare nanometer-sized emulsion particles. By changing the repeat number of treatments, the diameters of the emulsion particles became different. The above nano-emulsions were stirred and dichloromethane was extracted out to harden the nano-emulsion. Dichloromethane used for dissolving the polymer and the active agent was extracted to the aqueous phase, and then evaporated out to the air. After removing the dichloromethane, emulsion of PMMA polymers are hardened and become nano-particles. The above hardened nano-particles are purified by removing the surfactants with dialysis. As a result, PMMA polymers nano particles containing co-Q10 as active agents with predetermined diameters were obtained.

Quantitative analysis of the contents of the active agents contained in the nano particles purified by dialysis was proceeded by liquid chromatography. The contents of the active agents were controlled to be 2% by dilution or concentration. Concentration was practiced by removing water and moisture with reverse osmosis. Dilution was practiced by adding distilled water with a volume ratio.

Further, same processes were practiced using retinol and resveratrol to prepare nano particles.

Diameter distribution of the nano particles prepared in the above example was measured by dynamic laser light scattering method (Zetasizer 3000HS, Malvern, UK). Scattering angle was fixed at 90°, and temperature was fixed at 25°C. The relationship between the diameter of the particle and polydispersity was calculated by "contin" method.

The results are shown in Table 1.

**Table 1**

| | Active agent | Repeat number of MF treatment | Contents of Active agent | Average diameter |
|---|---|---|---|---|
| Example 1 | Retinol | 1 | 2.0% | 120nm |
| Example 2 | Retinol | 2 | 2.0% | 80nm |
| Example 3 | Retinol | 3 | 2.0% | 50nm |
| Example 4 | Co-Q10 | 1 | 2.0% | 120nm |
| Example 5 | Co-Q10 | 2 | 2.0% | 80nm |
| Example 6 | Co-Q10 | 3 | 2.0% | 50nm |
| Example 7 | Resveratrol | 1 | 2.0% | 120nm |
| Example 8 | Resveratrol | 2 | 2.0% | 80nm |
| Example 9 | Resveratrol | 3 | 2.0% | 50nm |

In table 1, the MF means microfluidizer.

According to above results, it was preferable to repeat the microfluidizer treatment 3 times to prepare nano particles with diameters of about 50nm. In this case, solid component contained is 5.17%, wherein PMMA polymer was 3.17% and active agent co-Q10 was 2.00%. The above products were used in percutaneous absorption test and formulation test.

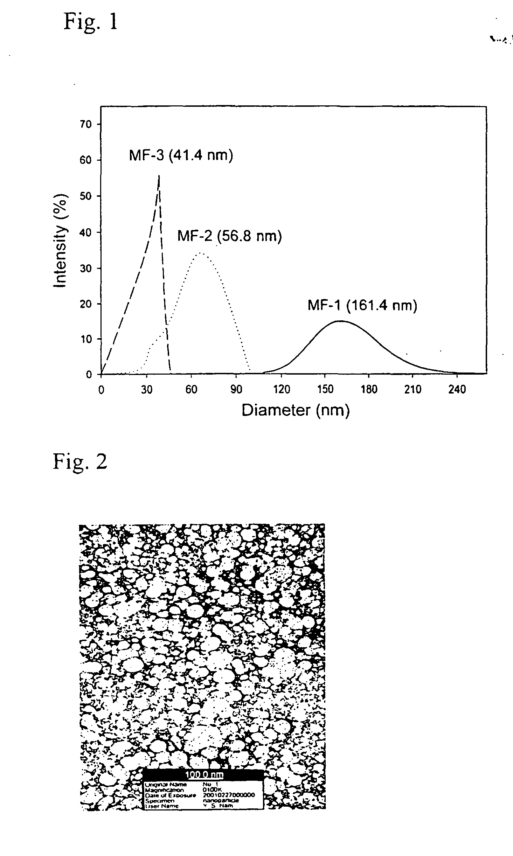

As examples for the results, the testing results of Examples 1 to 3 are shown in Fig. 1. From the above results, we found that as the number of the treatments with the microfluidizer increased, average diameters of the nano-particles prepared become smaller and diameter distribution becomes narrower. Therefore, it is clear that expected sizes of nano particles are prepared by controlling the treatments with the microfluidizer.

### Examples 10 to 18

PMMA (molecular weight 75,000) was used as a polymer, and retinol, co-Q10 and resveratrol were used as active agents. Each of 0.25g of co-Q10 as an active agent was dissolved in three flasks with 56ml of dichloromethane, then 0.5g, 1g and 2g of PMMA were added to each of the above flask to prepare phase 1. 4g of PEG 60 hydrogenated castor oil was homogeneously dissolved in each of the flasks. Then, 4500ml of distilled water was added thereto. This method has the advantage of leading to a spontaneous preparation of nano-particles, which uses the general solubilization. Dichloromethane was removed from phase 1 by solvent-extraction. Then, Nano-particles were diluted and concentrated to quantitatively arrange the contents of the active agents contained therein to 2%. Each of the nano particles containing retinol and resveratrol were prepared in the same manner. The results are shown in Table 2.

**Table 2**

| | Active agent | Contents of the PMMA (g) | Contents of Active agent | Average diameter |
|---|---|---|---|---|
| Example 10 | Retinol | 2.0 | 2.0% | 122nm |
| Example 11 | Retinol | 1.0 | 2.0% | 81 nm |
| Example 12 | Retinol | 0.5 | 2.0% | 45nm |
| Example 13 | Co-Q10 | 2.0 | 2.0% | 132nm |
| Example 14 | Co-Q10 | 1.0 | 2.0% | 89nm |
| Example 15 | Co-Q10 | 0.5 | 2.0% | 45nm |
| Example 16 | Resveratrol | 2.0 | 2.0% | 125nm |
| Example 17 | Resveratrol | 1.0 | 2.0% | 89nm |
| Example 18 | Resveratrol | 0.5 | 2.0% | 57nm |

### Examples 19 to 27

PMMA (molecular weight 75,000) was used as a polymer, and retinol, co-Q10 and resveratrol were used as active agents. Each of 0.25g of co-Q10 as an active agent was dissolved in three flasks with 56ml of dichloromethanes, then 0.5g, 1g and 2g of PMMA were added to each of the flasks to prepare phase 1. 4g of PEG 60 hydrogenated castor oil was homogeneously dissolved in each of the flasks. Then, 4500ml of distilled water was added thereto to prepare the nano-particle emulsions. Organic solvent of phase 1 was removed by spray drier system, and water was also removed. Active agent contained in white powder obtained therein was quantitatively analyzed to make the contents of the active agents unique, then the resultant powders were re-dispersed to quantitatively arrange the contents of the active agents contained therein to 2%, which were used in the following tests.

In this case, the sizes of nano particles were very different according to the preparation conditions. In the present examples, flow rate was 05ml/min, temperature of the drier was 150°C and carrier gas flow was 100ml/min. Nano particles pass through the drier with the carrier gas were filtered using the membrane filter, which is different from the conventional spray drier. Each of the nano particles containing retinol and resveratrol were prepared with the same manner. The results are shown Table 3.

**Table 3**

| | Active agent | Contents of the PMMA (g) | Contents of Active agent | Average diameter |
|---|---|---|---|---|
| Example 19 | Retinol | 2.0 | 2.0% | 150nm |
| Example 20 | Retinol | 1.0 | 2.0% | 96nm |
| Example 21 | Retinol | 0.5 | 2.0% | 44nm |
| Example 22 | Co-Q10 | 2.0 | 2.0% | 143nm |
| Example 23 | Co-Q10 | 1.0 | 2.0% | 94nm |
| Example 24 | Co-Q10 | 0.5 | 2.0% | 51nm |
| Example 25 | Resveratrol | 2.0 | 2.0% | 145nm |
| Example 26 | Resveratrol | 1.0 | 2.0% | 99nm |
| Example 27 | Resveratrol | 0.5 | 2.0% | 42nm |

### Formulation

Hereafter, various formulations using the nano particles prepared in Examples 1 to 27 are described. In the following table, items referred to as "Example" indicates the nano-particle prepared in the example.

### Formulations 1 to 9: nutritive cream

Formulations 1 to 9 describe nutrient cream formulation, shown in Table 4.

**Table 4**

| Composition | Form 1 | Form 2 | Form 3 | Form 4 | Form 5 | Form 6 | Form 7 | Form 8 | Form 9 |
|---|---|---|---|---|---|---|---|---|---|
| wax | 2.0 | 2.0 | 2.0 | 2.0 | 2.0 | 2.0 | 2.0 | 2.0 | 2.0 |
| glycerolstearate | 2.5 | 2.5 | 2.5 | 2.5 | 2.5 | 2.5 | 2.5 | 2.5 | 2.5 |
| cetostearate | 1.5 | 1.5 | 1.5 | 1.5 | 1.5 | 1.5 | 1.5 | 1.5 | 1.5 |
| polysolbate 60 | 0.5 | 0.5 | 0.5 | 0.5 | 0.5 | 0.5 | 0.5 | 0.5 | 0.5 |
| solvitancesquiolate | 5.0 | 5.0 | 5.0 | 5.0 | 5.0 | 5.0 | 5.0 | 5.0 | 5.0 |
| cethylethylhexanoate | 5.0 | 5.0 | 5.0 | 5.0 | 5.0 | 5.0 | 5.0 | 5.0 | 5.0 |
| squalan | 8.0 | 8.0 | 8.0 | 8.0 | 8.0 | 8.0 | 8.0 | 8.0 | 8.0 |
| flux paraffin | 8.0 | 8.0 | 8.0 | 8.0 | 8.0 | 8.0 | 8.0 | 8.0 | 8.0 |
| glycerin | 4.0 | 4.0 | 4.0 | 4.0 | 4.0 | 4.0 | 4.0 | 4.0 | 4.0 |
| propyleneglycol | 5.0 | 5.0 | 5.0 | 5.0 | 5.0 | 5.0 | 5.0 | 5.0 | 5.0 |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| plant extract | small | small | small | small | small | small | small | small | small |
| antiseptics | small | small | small | small | small | small | small | small | small |
| dye | small | small | small | small | small | small | small | small | small |
| fragrant | small | small | small | small | small | small | small | small | small |
| distilled water | to 100 | to 100 | to 100 | to 00 | to 100 | to 100 | to 100 | to 100 | to 100 |

In the table "Form" means formulation.

### Formulations 10 to 18: moisturizing preparation

Formulations 10 to 19 describe moisturizing preparation, shown in Table 5.

**Table 5**

| Composition | Form 10 | Form 11 | Form 12 | Form 13 | Form 14 | Form 15 | Form 16 | Form 17 | Form 18 |
|---|---|---|---|---|---|---|---|---|---|
| Cetylethylhexanoate | 5.0 | 5.0 | 5.0 | 5.0 | 5.0 | 5.0 | 5.0 | 5.0 | 5.0 |
| cetostearylalcohol | 1.0 | 1.0 | 1.0 | 1.0 | 1.0 | 1.0 | 1.0 | 1.0 | 1.0 |
| lipophilicmonostearic acid stearate | 0.8 | 0.8 | 0.8 | 0.8 | 0.8 | 0.8 | 0.8 | 0.8 | 0.8 |
| scualan | 2.0 | 2.0 | 2.0 | 2.0 | 2.0 | 2.0 | 2.0 | 2.0 | 2.0 |
| polysolbate 60 | 1.5 | 1.5 | 1.5 | 1.5 | 1.5 | 1.5 | 1.5 | 1.5 | 1.5 |
| solbitancesquiolate | 0.5 | 0.5 | 0.5 | 0.5 | 0.5 | 0.5 | 0.5 | 0.5 | 0.5 |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| glycerin | 8.0 | 8.0 | 8.0 | 8.0 | 8.0 | 8.0 | 8.0 | 8.0 | 8.0 |
| trimethanol amine | 0.2 | 0.2 | 0.2 | 0.2 | 0.2 | 0.2 | 0.2 | 0.2 | 0.2 |
| carboxyvinyl polymer | 0.2 | 0.2 | 0.2 | 0.2 | 0.2 | 0.2 | 0.2 | 0.2 | 0.2 |
| antisepices | small | small | small | small | small | small | small | small | small |
| dye | small | small | small | small | small | small | small | small | small |
| fragrant | small | small | small | small | small | small | small | small | small |
| distilled water | to 100 | to 100 | to 100 | to 100 | to 100 | to 100 | to 100 | to 100 | to 100 |

### Formulations 19 to 27: skin freshener

Formulations 19 to 27 describe skin freshener formulation, shown in Table 6.

**Table 6**

| Composition | Form 19 | Form 20 | Form 21 | Form 22 | Form 23 | Form 24 | Form 25 | Form 26 | Form 27 |
|---|---|---|---|---|---|---|---|---|---|
| betain | 3.0 | 3.0 | 3.0 | 3.0 | 3.0 | 3.0 | 3.0 | 3.0 | 3.0 |
| natto gum | 3.0 | 3.0 | 3.0 | 3.0 | 3.0 | 3.0 | 3.0 | 3.0 | 3.0 |
| cellulose gum | 0.08 | 0.08 | 0.08 | 0.08 | 0.08 | 0.08 | 0.08 | 0.08 | 0.08 |
| ethanol | 5.0 | 5.0 | 5.0 | 5.0 | 5.0 | 5.0 | 5.0 | 5.0 | 5.0 |
| polyoxyethylene | 0.5 | 0.5 | 0.5 | 0.5 | 0.5 | 0.5 | 0.5 | 0.5 | 0.5 |
| rigid castor oil | | | | | | | | | |
| acetictocopherol | 0.2 | 0.2 | 0.2 | 0.2 | 0.2 | 0.2 | 0.2 | 0.2 | 0.2 |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| anticeptics | small | small | small | small | small | small | small | small | small |
| dye | small | small | small | small | small | small | small | small | small |
| distilled water | to 100 | to 100 | to 100 | to 100 | to 100 | to 100 | to 100 | to 100 | to 100 |

### Formulations 28 to 36: gel

Formulations 28 to 36 describe gel formulation, shown in Table 7.

**Table 7**

| Composition | Form 28 | Form 29 | Form 30 | Form 31 | Form 32 | Form 33 | Form 34 | Form 35 | Form 36 |
|---|---|---|---|---|---|---|---|---|---|
| disodiumethylened iaminetetraacetate | 0.02 | 0.02 | 0.02 | 0.02 | 0.02 | 0.02 | 0.02 | 0.02 | 0.02 |
| etoxyglycol | 1.00 | 1.00 | 1.00 | 1.00 | 1.00 | 1.00 | 1.00 | 1.00 | 1.00 |
| polyacrylate | 20.00 | 20.00 | 20.00 | 20.00 | 20.00 | 20.00 | 20.00 | 20.00 | 20.00 |
| ethanol | 30.00 | 30.00 | 30.00 | 30.00 | 30.00 | 30.00 | 30.00 | 30.00 | 30.00 |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| hydrogenated castor oil | 0.80 | 0.80 | 0.80 | 0.80 | 0.80 | 0.80 | 0.80 | 0.80 | 0.80 |
| phenyltrimethcon | 0.20 | 0.20 | 0.20 | 0.20 | 0.20 | 0.20 | 0.20 | 0.20 | 0.20 |
| triethanolamine | 0.40 | 0.40 | 0.40 | 0.40 | 0.40 | 0.40 | 0.40 | 0.40 | 0.40 |
| fragrant | small | small | small | small | small | small | small | small | small |
| distilled water | to 100 | to 100 | to 100 | to 100 | to 100 | to 100 | to 100 | to 100 | to 100 |

### Formulations 37 to 45: spray

Formulations 37 to 45 describe spray formulation, shown in Table 8.

**Table 8**

| Composition | Form 64 | Form 65 | Form 66 | Form 67 | Form 68 | Form 69 | Form 70 | Form 71 | Form 72 |
|---|---|---|---|---|---|---|---|---|---|
| triethanolamine | 0.2 | 0.2 | 0.2 | 0.2 | 0.2 | 0.2 | 0.2 | 0.2 | 0.2 |
| polyvinylpyrrolidon/ | 3.0 | 3.0 | 3.0 | 3.0 | 3.0 | 3.0 | 3.0 | 3.0 | 3.0 |
| vinylacetat | | | | | | | | | |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| glycerine | 5.0 | 5.0 | 5.0 | 5.0 | 5.0 | 5.0 | 5.0 | 5.0 | 5.0 |
| polyacrylate | 0.2 | 0.2 | 0.2 | 0.2 | 0.2 | 0.2 | 0.2 | 0.2 | 0.2 |
| distilled water | to 100 | to 100 | to 100 | to 100 | to 100 | to 100 | to 100 | to 100 | to 100 |

### Formulations 46 to 54: ointment

Formulations 46 to 54 describes ointment formulation, shown in Table 9.

**Table 9**

| Compostion | Form 46 | Form 47 | Form 48 | Form 49 | Form 50 | Form 51 | Form 52 | Form 53 | Form 54 |
|---|---|---|---|---|---|---|---|---|---|
| caprin/capryltglyceride | 10.0 | 10.0 | 10.0 | 10.0 | 10.0 | 10.0 | 10.0 | 10.0 | 10.0 |
| liquid paraffin | 10.0 | 10.0 | 10.0 | 10.0 | 10.0 | 10.0 | 10.0 | 10.0 | 10.0 |
| solbitancesquioliate | 6.0 | 6.0 | 6.0 | 6.0 | 6.0 | 6.0 | 6.0 | 6.0 | 6.0 |
| octyldodeces-25 | 9.0 | 9.0 | 9.0 | 9.0 | 9.0 | 9.0 | 9.0 | 9.0 | 9.0 |
| cethylehtylhexanoate | 10.0 | 10.0 | 10.0 | 10.0 | 10.0 | 10.0 | 10.0 | 10.0 | 10.0 |
| squalin | 1.0 | 1.0 | 1.0 | 1.0 | 1.0 | 1.0 | 1.0 | 1.0 | 1.0 |
| salicylic acid | 1.0 | 1.0 | 1.0 | 1.0 | 1.0 | 1.0 | 1.0 | 1.0 | 1.0 |
| glycerin | 15.0 | 15.0 | 15.0 | 15.0 | 15.0 | 15.0 | 15.0 | 15.0 | 15.0 |
| solibitol | 10.0 | 10.0 | 10.0 | 10.0 | 10.0 | 10.0 | 10.0 | 10.0 | 10.0 |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| distilled water | to 100 | to 100 | to 100 | to 100 | to 100 | to 100 | to 100 | to 100 | to 100 |

### Formulations 55 to 63: patch

Formulations 55 to 63 describe patch formulation, shown in Table 10.

**Table 10**

| Composition | Form 55 | Form 56 | Form 57 | Form 58 | Form 59 | Form 60 | Form 61 | Form 62 | Form 63 |
|---|---|---|---|---|---|---|---|---|---|
| polyvinylalcohol | 2.0 | 2.0 | 2.0 | 2.0 | 2.0 | 2.0 | 2.0 | 2.0 | 2.0 |
| polyvinyl pyrrolidon | 3.0 | 3.0 | 3.0 | 3.0 | 3.0 | 3.0 | 3.0 | 3.0 | 3.0 |
| sodium polyacrylic acid | 3.0 | 3.0 | 3.0 | 3.0 | 3.0 | 3.0 | 3.0 | 3.0 | 3.0 |
| sodium algenate | 1.0 | 1.0 | 1.0 | 1.0 | 1.0 | 1.0 | 1.0 | 1.0 | 1.0 |
| retinylpalmitate | 1.0 | 1.0 | 1.0 | 1.0 | 1.0 | 1.0 | 1.0 | 1.0 | 1.0 |
| butyleneglycol | 3.0 | 3.0 | 3.0 | 3.0 | 3.0 | 3.0 | 3.0 | 3.0 | 3.0 |
| chondroitin sulfate | 1.0 | 1.0 | 1.0 | 1.0 | 1.0 | 1.0 | 1.0 | 1.0 | 1.0 |
| *Schizophyllum coummune* extract | 1.0 | 1.0 | 1.0 | 1.0 | 1.0 | 1.0 | 1.0 | 1.0 | 1.0 |
| medofoam oil | 1.0 | 1.0 | 1.0 | 1.0 | 1.0 | 1.0 | 1.0 | 1.0 | 1.0 |
| PEG(20) solbitanstearate | 1.0 | 1.0 | 1.0 | 1.0 | 1.0 | 1.0 | 1.0 | 1.0 | 1.0 |
| BHT | small | small | small | small | small | small | small | small | small |
| Zinc oxide | small | small | small | small | small | small | small | small | small |
| Example 3 | 5.0 | - | - | - | - | - | - | - | - |
| Example 6 | - | 5.0 | - | - | - | - | - | - | - |
| Example 9 | - | - | 5.0 | - | - | - | - | - | - |
| Example 12 | - | - | - | 5.0 | - | - | - | - | - |
| Example 15 | - | - | - | - | 5.0 | - | - | - | - |
| Example 18 | - | - | - | - | - | 5.0 | - | - | - |
| Example 21 | - | - | - | - | - | - | 5.0 | - | - |
| Example 24 | - | - | - | - | - | - | - | 5.0 | - |
| Example 27 | - | - | - | - | - | - | - | - | 5.0 |
| distilled water | to 100 | to 100 | to 100 | to 100 | to 100 | to 100 | to 100 | to 100 | to 100 |

### Test Example 1: observation of the nano particles with transmission electron microscopy

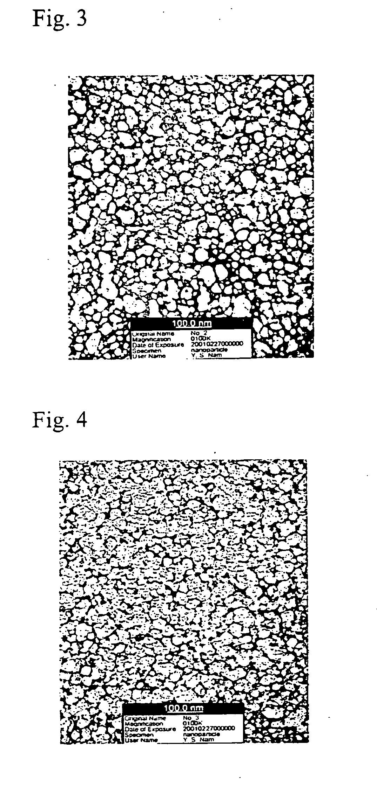

The diameter distribution and characteristics of the nano particles are observed with transmission electron microscopy. The nano particles are dispersed in triply distilled water, dyed with 1% of uranyl acetate, dried for 30 minute, and then observed. Results obtained by observing Examples 4 to 6 are shown in Figs. 2 to 4, which show the results treated by the microfluidizer 1 to 3 times respectively.

From the results, it was found that diameters of the nano particles observed with transmission electron microscopy are same as those observed with laser light scattering method, and that the diameters of the nano particles are controlled by controlling the treating times of the microfluidizer.

### Test Example 2: stability test of the active agents contained in nano particles

The stability of the active agents contained in nano particles was observed when they were stored for a long time.

Nano particle samples obtained in each of the examples were stored in thermostatic baths with the temperatures of 0°C, 25°C and 45°C, then the amounts of the active agents were measured in predetermined intervals. For example, the results of Examples 1 to 9 are shown in Table 11. In the table, the initial amount of the active agent is regarded as 100, then the relative amount of the active agent remaining with time is calculated.

Further, the same measurements were practiced to Formulations 1 to 3, 10 to 12 and 46 to 48, and the results are shown in Table 12. In the above measurements, it was found that the active agents contained in the nano-particles are preserved as safe and stable.

**Table 11**

| | Condition | 7days | 15days | 30days | 45days |
|---|---|---|---|---|---|
| Example | 0°C | 100% | 100% | 98% | 95% |
| | 1 25°C | 100% | 94% | 90% | 87% |
| | 45°C | 100% | 91% | 86% | 83% |
| Example 2 | 0°C | 100% | 100% | 99% | 97% |
| | 25°C | 100% | 95% | 92% | 89% |
| | 45°C | 100% | 92% | 87% | 84% |
| Example 3 | 0°C | 100% | 100% | 99% | 96% |
| | 25°C | 100% | 96% | 92% | 88% |
| | 45°C | 100% | 93% | 88% | 84% |
| Example 4 | 0°C | 100% | 100% | 100% | 100% |
| | 25°C | 100% | 99% | 95% | 91% |
| | 45°C | 100% | 94% | 90% | 85% |
| Example 5 | 0°C | 100% | 100% | 100% | 100% |
| | 25°C | 100% | 99% | 93% | 85% |
| | 45°C | 100% | 95% | 89% | 83% |
| Example 6 | 0°C | 100% | 100% | 100% | 100% |
| | 25°C | 100% | 99% | 92% | 81% |
| | 45°C | 100% | 96% | 86% | 80% |
| Example 7 | 0°C | 100% | 100% | 97% | 96% |
| | 25°C | 100% | 95% | 91% | 82% |
| | 45°C | 100% | 90% | 85% | 79% |
| Example 8 | 0°C | 100% | 100% | 97% | 96% |
| | 25°C | 100% | 96% | 94% | 92% |
| | 45°C | 100% | 91% | 87% | 85% |
| Example 9 | 0°C | 100% | 100% | 98% | 96% |
| | 25°C | 100% | 97% | 95% | 91% |
| | 45°C | 100% | 92% | 88% | 85% |

**Table 12**

| | Condition | 7days | 15days | 30days | 45days |
|---|---|---|---|---|---|
| Formulation 1 | 0°C | 100% | 100% | 98% | 96% |
| | 25°C | 100% | 97% | 95% | 91% |
| | 45°C | 100% | 92% | 88% | 85% |
| Formulation 2 | 0°C | 100% | 100% | 100% | 100% |
| | 25°C | 100% | 99% | 92% | 81% |
| | 45°C | 100% | 96% | 86% | 80% |
| Formulation 3 | 0°C | 100% , | 100% | 97% | 96% |
| | 25°C | 100% | 95% | 91% | 82% |
| | 45°C | 100% | 90% | 85% | 79% |
| Formulation 10 | 0°C | 100% | 100% | 97% | 96% |
| | 25°C | 100% | 95% | 91% | 82% |
| | 45°C | 100% | 90% | 85% | 79% |
| Formulation 11 | 0°C | 100% | 100% | 97% | 96% |
| | 25°C | 100% | 96% | 94% | 92% |
| | 45°C | 100% | 91% | 87% | 85% |
| Formulation 12 | 0°C | 100% | 100% | 99% | 97% |
| | 25°C | 100% | 95% | 92% | 89% |
| | 45°C | 100% | 92% | 87% | 84% |
| Formulation 46 | 0°C | 100% | 100% | 99% | 96% |
| | 25°C | 100% | 96% | 92% | 88% |
| | 45°C | 100% | 93% | 88% | 84% |
| Formulation 47 | 0°C | 100% | 100% | 97% | 96% |
| | 25°C | 100% | 96% | 94% | 92% |
| | 45°C | 100% | 91% | 87% | 85% |
| Formulation 48 | 0°C | 100% | 100% | 98% | 96% |
| | 25°C | 100% | 97% | 95% | 91% |
| | 45°C | 100% | 92% | 88% | 85% |

From the above tests, retinol, co-Q10 and resveartrol contained in the nano particles are stable when stored for a long time. Stabilities were not influenced by the size of the nano particles. Active agents were stable for a long time in lower temperature, but in the thermostatic bath with 45°C, the contents of the active agents became less after 15 days. In the above test, the nano particles were stable, because the hydrophobic PMMA polymers prevented the water from being dispersed into the nano-particles and therefore inner active agents did not come in contact with the water. Active agents contained in the hydrophobic polymer are buried in the polymer chain, and the passages for the water to disperse are blocked, and therefore, contacts are strictly restricted. Compared with the present invention, conventional micro capsule type particles are not stable, because there are many big passages for water to penetrate into the particle. But such defaults can be solved by present percutaneous systems, )

### Test Example 3: percutaneous absorption test according to the size of nano particles

Nano particles with diameters of 50nm, 80nm, 120nm and 150nm using PMMA polymer attached by fluorescent material were prepared respectively, then applied to the skin of an animal (female atrichia(hairless) guinea-pig). After a predetermined time, the skin was cut and fluorescent emitted from the nano particles of the skin was measured.

### 1) Preparation of the fluorescent PMMA polymer nano particles

Fluorescent molecules are covalently bonded to carboxyl group of the methacrylate in polymethylmetharylate-co-methacrylate (methylmetharylate : methacrylate = 84 : 16 in molecular ratio). They were prepared as follows. 34g of polymethylmetharylate-co-methacrylate was dissolved in 150g of dehydrated methylenechloride, then 132mg of dicydlohexylcarbodiimide and 74mg of N-hydroxysuccinimide was added to activate carboxyl group. After 1 hour of stirring, 222mg of fluoresceinamine was added thereto. Fluorescent molecule forms a covalent bonding with polymer chain by forming amide bonding between first amine of the fluorescein and activated carboxyl group. Reaction was performed in a dark room for 5 hours. A by product, dicyclohexylurea, was removed by nylon filter, and the reaction product was deposited into the diethylether to remove the reaction agent and residue, then was reserved in the triply distilled water for a day to remove the residue, and dried in a vacuum oven. PMMA nano-particles to which fluorescent materials were attached and having diameters of 50nm, 80nm, 120nm and 150nm were prepared.

### 2) Percutaneous absorption test according to the size of nano particles

8-week old female atrichia(hairless) guinea pig (strain IAF/HA-hrBR) was used in the present test. Abdominal portion of the guinea pig was cut and applied to Franz- type diffusion cell (Lab Instruments, Korea).

50nM of phosphate buffer solution (pH 7.4, 0.1M NaCl) was added to the vessels (5 ml) for Franz-type diffusion cell. The diffusion cell was mixed and dispersed with 600 rpm at 32°C, and 50 µl of solution in which 10%(w/v) of the fluorescent PMMA nano particles prepared in each particle size was added to each donor vessel.

Absorption was practiced for a predetermined time (12 hours), and the area for absorption was controlled to be 0.64m². After absorption and dispersion was performed, residue of the nano particle on the skin was washed by 10 ml of Kimwipes and ethanol. Then, the skin was cut and the distribution of the PMMA nano particles absorbed into the skin was measured.

### 3) Measurement using Confocal Laser Scannin Microscopy (CLMS)

To measure the percutaneous absorption of the fluorescent PMMA nano particles, confocal laser scannin nicroscopy (CLMS) was used. Ar/Kr laser source was mounted in the CLMS, and Nikkon eclipse TE300 was used together with oil- immersed Plan apo 60X1.4 Na objective lens. Each of the samples were measured along the z-axis.

### 4) Result

The results of the skin distribution measurement for nano-particles are shown in Table 13.

**Table 13**

| Average particle size | Penetrating delpth | Reference |
|---|---|---|
| 50nm | 30µm | Fig. 5 |
| 80nm | 18µm | Fig. 6 |
| 120nm | 12µm | |
| 500nm | 6µm | |

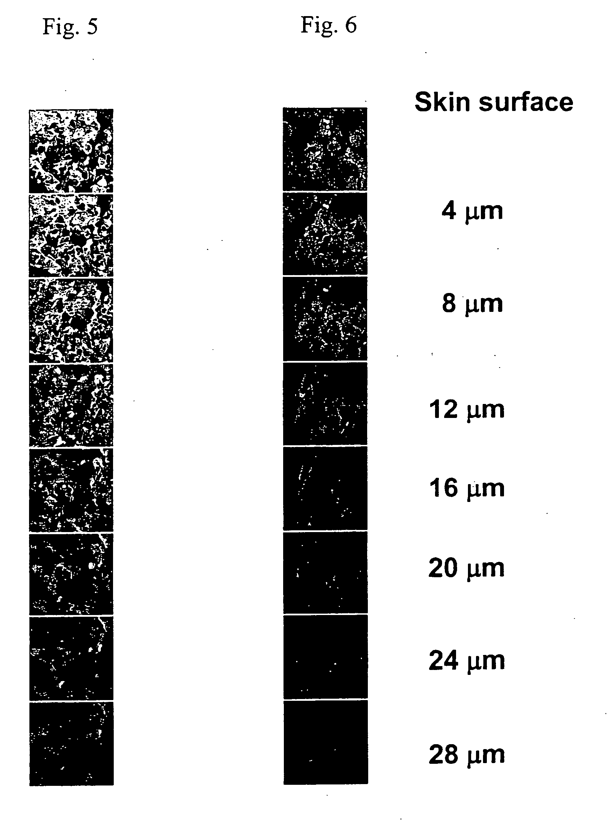

Distributions for particles of 50nm and 80nm are shown in Fig. 5 and Fig. 6.

From the above tests, it was found that percutaneous absorption of the nano particles depend on the size of the particle. Nano particles with 50nm of diameter penetrated the epidermis to the upper layer of the dermis. Nano particles with 500nm of diameter could not penetrate the epidermis, but just stayed on the skin. Nano particles with diameters of about 50nm disperse into the lipid between the skin cells, and the hydrophobicity of the polymer promotes the absorption. Further, even though active agents that are not stable or hard enough to be absorbed can penetrate the skin by being contained into the nano particles with diameters of about 50nm.

### Test Example 4: Measurement for percutaneous absorption of the retinol

Percutaneous absorption for the nano particles containing retinol prepared in Examples 1 to 3, 10 to 12 and 19 to 21 and Formulations 1, 10, 19, 28 and 46 were measured.

### 1) Method

8-week old female atrichia guinea pig (strain IAF/HA-hrBR) was used in the present test. Abdominal portion of the guinea pig was cut and applied to Franz-type diffusion cell (Lab Instruments, Korea). 50nM of phosphate buffer solution (pH 7.4, 0.1M NaCl) was added to the vessels (5 ml) for Franz-type diffusion cell. The diffusion cell was mixed and dispersed with 600 rpm at 32°C, and 50 µl of solution in which 10%(w/v) of the PMMA nano particles containing retinol was added to each donor vessel.

Absorption was practiced for a predetermined time, and the area for absorption was controlled to be 0.64m². After absorption and dispersion were performed, residue of the nano particle on the skin was washed by 10 ml of Kimwipes and ethanol. Then, the skin was cut and ground by tip type homogenizer (Polytron PT2100. Switzerland), then the retinol absorbed was extracted by using 4ml of dichloromethane. The extract was filtered by the 4.5µm of nylon membrane, then the content was measured by the following HPLC.

ODO(capric/caprylic triglyceride)s with 2% of retinol and 1% of retinol were use as control group.

### 2) Analysis condition

a. column: C18 (4.6X250mm, 5m)
b. moving phase: methanol or ethanol 93%
c. flow rate: 0.8ml/min
d. detector: UV 325nm

### 3) Result

Results are shown in Table 14. In the table, ODO means capric/caprylic triglyceride.

**Table 14**

| | | Percutaneous absorption (µg) | Increasing rate of percutaneous absorption |
|---|---|---|---|
| Control (2% of retinol in ODO) | | 11.5 (±0.1) | - |
| | Example 1 | 12.5 (±2.4) | 1.1 |
| | Example 2 | 12.7 (±5.3) | 1.1 |
| | Example 3 | 65.4 (±5.5) | 5.7 |
| | Example 10 | 13.5(±3.1) | 1.2 |
| | Example 11 | 15.7(±4.2) | 1.4 |
| | Example 12 | 75.4(±5.2) | 6.6 |
| | Example 19 | 16.5(±2.1) | 1.4 |
| | Example 20 | 32.7(±3.2) | 2.8 |
| | Example 21 | 65.4(±6.6) | 5.7 |
| Control (0.1% retinol in ODO) | | 0.42 | - |
| | Form 1 | 2.5 | 5.9 |
| | Form 10 | 2.6 | 6.2 |
| | Form 19 | 2.8 | 6.7 |
| | Form 28 | 2.9 | 6.9 |
| | Form 46 | 2.4 | 5.7 |

As seen from the above results, in Example 1 to 3 which contain retinol, Example 1 with average particle 120 nm showed an increase in the percutaneous absorption compared to the control group. Example 2 with average particle 80 nm also showed good percutaneous absorption. Further, Example 3 with average particle size of 50 nm showed an increase of 5.7 times in percutaneous absorption compared to the control group. In Example 3, it was found that percutaneous absorption increases as the size of the nano particle decreases.

### Test Example 5: Measurement for percutaneous absorption of the co-Q10

Percutaneous absorption for the nano particles containing co-Q10 prepared in Examples 4 to 6, 13 to 15 and 22 to 24 and Formulation 2, 11, 20, 29 and 47 were measured.

### 1) Method

Same process executed in above Test Example 4 was performed, except that co-Q10 was used as an active agent. Control group was also the same.

### 3) Analysis condition

a. column: Ubondapak C18 (3.9X150mm)
b. moving phase: methanol/ethanol (40/60)
c. flow rate: 1ml/min
d. detector: UV 275nm

### 4) Result

Results are shown in Table 15. In the table, ODO means capric/caprylic triglyceride.

**Table 15**

| | | Percutaneous absorption (µg) | Increasing rate of percutaneous absorption |
|---|---|---|---|
| Control (2% co-Q10 in ODO) | | 8.5 (±2.5) | - |
| | Example 4 | 8.9 (±3.2) | 1.0 |
| | Example 5 | 10.1 (±2.6) | 1.2 |
| | Example 6 | 52.1 (±5.7) | 6.1 |
| | Example 13 | 9.9 (±3.2) | 1.2 |
| | Example 14 | 12.3 (±2.6) | 1.4 |
| | Example 15 | 55.2 (±5.7) | 6.5 |
| | Example 22 | 8.9 (±3.2) | 1.0 |
| | Example 23 | 13.3 (±2.6) | 1.6 |
| | Example 24 | 57.9 (±5.7) | 6.8 |
| Control (0.1% co-Q10 in ODO) | | 0.5 | - |
| | Form 2 | 3.0 | 6.0 |
| | Form 11 | 3.4 | 6.8 |
| | Form 20 | 2.5 | 5.0 |
| | Form 29 | 2.8 | 5.6 |
| | Form 47 | 2.9 | 5.8 |

The results are much the same as those of Test Example 4, and show the same characterstices.

### Test Example 6: Measurement for percutaneous absorption of the resveratrol

Percutaneous absorption for the nano particles containing resveratrol prepared in examples 7 to 9, 16 to 18 and 25 to 27 and formulation examples 3, 9, 21, 30 and 48 was measured.

### 1) Method

Same process executed in above Test Example 4 was performed, except that resveratrol was used as an active agent. The control group was also the same.

### 2) Analysis condition

a. column: Ubondapak C18 (3.9X150mm)
b. moving phase: methanol/ethanol (40/60)
c. flow rate: 1ml/min
d. detector: UV 275nm

### 3) Result

Results are shown in Table 16.

**Table 16**

| | | Percutaneous absorption (µg) | Increasing rate of percutaneous absorption |
|---|---|---|---|
| Control (2% reveratrol in ODO) | | 6.2 (±1.9) | - |
| | Example 7 | 7.1 (±2.6) | 1.1 |
| | Example 8 | 6.5 (±2.4) | 1.0 |
| | Example 9 | 45.7 (±6.2) | 7.4 |
| | Example 16 | 7.9 (±3.1 ) | 1.3 |
| | Example 17 | 10.5 (±4.4) | 1.7 |
| | Example 18 | 48.2 (±8.2) | 7.8 |
| | Example 25 | 7.7 (±3.6) | 1.2 |
| | Example 26 | 9.5 (±2.9) | 1.5 |
| | Example 27 | 50.4 (±7.2) | 8.1 |
| Control (0.1% reveratrol in ODO) | | 0.3 | - |
| | Form 3 | 2.8 | 9.3 |
| | Form 9 | 2.5 | 8.3 |
| | Form 21 | 2.5 | 8.3 |
| | Form 30 | 2.7 | 9.0 |
| | Form 48 | 2.6 | 8.7 |

The results are much the same as those of Test Examples 4 and 5, and show the same characterstices.

### Test Example 7: Measurement for skin irritation

According to the closed patch test method, samples from the examples were applied to healthy men and women on their forearm once a day, and capped with plastic to prevent contact with outer atmosphere, then the degree of the irritation was measured after 1 day, 3 days and 7 days.

The degree of the irritation was determined according to the following Table 17.

**Table 17**

| Value | Degree of the irritation |
|---|---|
| 0 | Nothing |
| 1 | Least irritation (very slight) |
| 2 | Some irritation (erythema) |
| 3 | Intense irritation (erythema, edema) |
| 4 | Very intense irritation (erythema, edema) |

Average degree value of the irritation was calculated by summing up the degree of the each person then dividing the sum with the number of the persons. The results are shown in Table 18.

**Table 18**

| Sample No. | Degree of the irritation (%) | | | Sample No. | Degree of the irritation (%) | | |
|---|---|---|---|---|---|---|---|
| | 1 day | 3 day | 7 day | | 1 day | 3 day | 7 day |
| Example 1 | 0.3 | 0.3 | 0.4 | Form 13 | 0.3 | 0.3 | 0.4 |
| Example 2 | 0.2 | 0.3 | 0.5 | Form 14 | 0.2 | 0.3 | 0.5 |
| Example 3 | 0.5 | 0.6 | 0.8 | Form 15 | 0.5 | 0.6 | 0.8 |
| Example 4 | 0.3 | 0.4 | 0.6 | Form 25 | 0.3 | 0.4 | 0.6 |
| Example 5 | 0.5 | 0.5 | 0.8 | Form 26 | 0.5 | 0.5 | 0.8 |
| Example 6 | 0.3 | 0.3 | 0.3 | Form 27 | 0.3 | 0.3 | 0.3 |
| Example 7 | 0.2 | 0.6 | 0.5 | Form 28 | 0.2 | 0.6 | 0.5 |
| Example 8 | 0.5 | 0.4 | 0.3 | Form 29 | 0.5 | 0.4 | 0.3 |
| Example 9 | 0.3 | 0.5 | 0.2 | Form 30 | 0.3 | 0.5 | 0.2 |
| Example 12 | 0.6 | 0.6 | 0.7 | Form 40 | 0.5 | 0.4 | 0.6 |
| Example 15 | 0.5 | 0.7 | 0.7 | Form 41 | 0.3 | 0.5 | 0.5 |
| Example 18 | 0.5 | 0.5 | 0.6 | Form 42 | 0.4 | 0.5 | 0.6 |
| Example 21 | 0.8 | 0.8 | 0.9 | Form 52 | 0.1 | 0.4 | 0.5 |
| Example 24 | 0.7 | 0.6 | 0.8 | Form 53 | 0.2 | 0.3 | 0.4 |
| Example 27 | 0.5 | 0.6 | 0.7 | Form 54 | 0.5 | 0.6 | 0.8 |
| Form 1 | 0.6 | 0.5 | 0.8 | Form 55 | 0.3 | 0.3 | 0.6 |
| Form 2 | 0.5 | 0.5 | 0.6 | Form 56 | 0.2 | 0.4 | 0.8 |
| Form 3 | 0.3 | 0.5 | 0.7 | | | | |

In case of Examples 1 to 9, little irritation was felt. Further, in Examples 12, 15, 18, 21, 24 and 27, no significant irritation was felt. In addition, the cosmetic and medical compositions containing the active agents prepared in Examples 1 to 27 did not cause skin irritation.

From the results above, it is sure that the nano particles prepared in the present invention show high affinity to the skin, and can be absorbed into the skin without causing skin irritation.

The present invention provides a percutaneous releasing material and agent having such characteristics as high stability of the active agent in the formulation, high topical absorption rate, decreased irritation on the skin and increased tactile comfort, and provides an external application agent composition by using nanometer-sized polymer particles.

## Claims

1. Percutaneous releasing material preparable by using polymer nano-sized particles having diameters of 30 to 150 nm to contain and hold physiologically active agent;
wherein said polymer is polymethylmetacrylate (PMMA);
wherein said polymer particles containing physiologically active agent penetrates through stratum corneum to the exterior upper layer of dermis, then effuses the physiologically active agent into the skin while staying in the upper layer of dermis.

2. Percutaneous releasing material according to claim 1, wherein said physiologically active agent is at least one selected from the group consisting of antibiotics, antitumor agent, anti-inflammatory agent, antipyretic, analgesia, anti-edema agent, antitussive agent, expectorant, depressant, muscle relaxant, antiepileptic, anti-ulcer agent, anti-melancholia agent, anti-allergy agent, cardiotonic agent, anti-arrhythmic agent, vasodilatin, hypotensive agent, antidiabetic, homoeostasis agent, hormone, antioxidant, growing hair agent, hair tonic, gumboil agent (antimicrobial agent), whitening agent, crease resistant or disapproval agent, collagen synthesizing accelerant, membrane fortifier and moisturizing agent.

3. Percutaneous releasing material according to claim 1, wherein the content of said polymer particles in the aqueous solution is 0.0001 percent by weight to 90 percent by weight.

4. Percutaneous releasing material according to claim 1, wherein the content of said polymer particles in the aqueous solution is 0.1 percent by weight to 50 percent by weight.

5. An external application agent composition comprising the percutaneous releasing material according to claim 1 to 4.

6. An external application agent composition according to claim 5, wherein the content of said percutaneous releasing material is 0.0001 percent by weight to 50 percent by weight, relative to the total amount of the external application agent composition.

7. An external application agent composition according to claim 5, wherein the formulation of the agent composition is skin freshener, moisturizing preparation, massage cream, nutrient cream, pack, gel, skin-adhesive cosmetic, lipstick, make-up base, foundation, shampoo, rinse, body-cleanser, soap, toothpaste, mouth wash, formulations for hair growth, lotion, ointment, gel, cream, patch or spray.

8. A method for preparing the percutaneous releasing material of claim 1 comprising the steps of:
1) Preparing oil phase by stirring the polymer having molecular weight of 5,000 to 1,000,000 and physiologically active agent;
2) Preparing first emulsion by mixing said oil phase and distilled water, in which surfactant is dissolved;
3) Preparing second emulsion by emulsifying said first emulsion with emulsifier under the condition that pressure is 500 bar to 1,500 bar and flow rate is 20 ml/min to 150 ml/min; and
4) Hardening said second emulsion.

## Patentansprüche

1. Perkutan freisetzendes Material, das unter Verwendung von Polymer-Nanoteilchen mit Durchmessern von 30 bis 150 nm so hergestellt werden kann, dass es physiologisch aktiven Wirkstoff enthält und behält,
wobei das besagte Polymer Polymethylmethacrylat (PMMA) ist,
wobei die besagten, physiologisch aktiven Wirkstoff enthaltenden Polymerpartikel durch das Stratum corneum zu der äußeren oberen Hautschicht dringen, und dann den physiologisch aktiven Wirkstoff in die Haut freisetzen, während sie in der oberen Hautschicht verbleiben.

2. Perkutan freisetzendes Material gemäß Anspruch 1, bei dem der besagte physiologisch aktive Wirkstoff mindestens einer ist, der aus der aus Antibiotika, Antitumorwirkstoffen, entzündungshemmenden Wirkstoffen, fiebersenkenden Mitteln, Schmerzmitteln, Antiödemwirkstoffen, Husten lösenden Wirkstoffen, Schleim lösenden Wirkstoffen, Beruhigungsmitteln, Muskel entspannenden, Mitteln gegen Epilepsie, Wirkstoffen gegen Geschwüre, Wirkstoffen gegen Trübsinn, antiallergischen Wirkstoffen, herzstärkenden Wirkstoffen, Wirkstoffen gegen Herzrhythmusstörungen, gefäßerweiternden Wirkstoffen, Blutdruck senkenden Wirkstoffen, Wirkstoffen gegen Diabetes, homöostatischen Wirkstoffen, Hormonen, Antioxidantien, Haarwuchsmitteln, Haartoniken, Wirkstoffen gegen Zahngeschwüre (antimikrobielle Wirkstoffe), Bleichmitteln, Wirkstoffen gegen Hautfaltenbildung oder abweisenden Wirkstoffen, Beschleunigern der Kollagensynthese, Membranverstärkern und Feuchtigkeitswirkstoffen bestehenden Gruppe ausgewählt wird.

3. Perkutan freisetzendes Material gemäß Anspruch 1, bei dem der Gehalt an besagten Polymerpartikeln in wässriger Lösung 0,0001 Gew.-% bis 90 Gew.-% beträgt.

4. Perkutan freisetzendes Material gemäß Anspruch 1, bei dem der Gehalt an besagten Polymerpartikeln in wässriger Lösung 0,1 Gew.-% bis 50 Gew.-% beträgt.

5. Zusammensetzung zur äußerlichen Anwendung, die das perkutan freisetzende Material gemäß der Ansprüche 1 bis 4 umfasst.

6. Zusammensetzung zur äußerlichen Anwendung gemäß Anspruch 5 in der der Gehalt an dem besagten perkutan freisetzenden Material 0,0001 Gew.-% bis 50 Gew.-% bezogen auf die Gesamtmenge der Zusammensetzung zur äußerlichen Anwendung ist.

7. Zusammensetzung zur äußerlichen Anwendung gemäß Anspruch 5, in der die Formulierung der Wirkstoffzusammensetzung ein Erfrischungsmittel für die Haut, eine Feuchtigkeitszubereitung, Massagecreme, Nährcreme, Packung, Gel, an der Haut haftendes Kosmetikum, Lippenstift, Make-up-Grundlage, Grundierung, Shampoo, Spülung, Reinigungsmittel für den Körper, Seife, Zahnpasta, Mundwasser, Formulierungen für das Haarwachstum, Lotion, Salbe, Gel, Creme, Pflaster oder Spray ist.

8. Verfahren zur Herstellung des perkutan freisetzenden Materials gemäß Anspruch 1, das folgende Schritte umfasst:
1. Herstellung einer ölphase durch Rühren des Polymers mit einem Molgewicht von 5000 bis 1 000 000 und physiologisch aktivem Wirkstoff,
2. Herstellung einer ersten Emulsion durch Mischen der besagten Ölphase und destilliertem Wasser, in dem ein oberflächenaktiver Stoff gelöst ist,
3. Herstellung einer zweiten Emulsion durch Emulgieren der besagten ersten Emulsion mit einem Emulgator unter der Bedingung, dass der Druck zwischen 500 und 1500 bar beträgt und die Durchflussrate 20 mL/min bis 150 mL/min beträgt, und
4. Aushärten der besagten Emulsion.

## Revendications

1. Matériau à libération percutanée pouvant être préparé en utilisant des particules de dimension nanométrique de polymère ayant des diamètres de 30 à 150 nm pour contenir et conserver un agent physiologiquement actif ;
dans lequel ledit polymère est un polymétacrylate de méthyle (PMMA) ;
dans lequel lesdites particules de polymère contenant un agent physiologiquement actif pénètrent à travers la couche cornée jusqu'à la couche extérieure supérieure du derme, puis répandent l'agent physiologiquement actif dans l'intérieur de la peau tout en restant dans la couche supérieure du derme.

2. Matériau à libération percutanée selon la revendication 1,
dans lequel ledit matériau physiologiquement actif est au moins un matériau choisi dans le groupe consistant en les antibiotiques, les agents anti-tumoraux, les agents anti-inflammatoires, les antipyrétiques, les analgésiques, les agents anti-oedèmes, les agents antitussifs, les expectorants, les calmants, les myorelaxants, les anti-épileptiques, les agents anti-ulcères, les agents anti-mélancolie, les agents anti-allergiques, les agents cardiotoniques, les agents anti-arythmie, les vasodilatateurs, les agents hypotension, les antidiabétiques, les agents d'homoéostase, les hormones, les anti-oxydants, les agents de croissance capillaire, les toniques capillaires, les agents de gumboil (agents anti-microbiens), les agents blanchissants, les agents d'infroissabilité ou de désapprobation, les accélérateurs de synthèse de collagène, les agents de renforcement de membranes et d'humidification.

3. Matériau à libération percutanée selon la revendication 1,
dans lequel la teneur desdites particules de polymère dans la solution aqueuse est de 0,0001 pour cent en poids à 90 pour cent en poids.

4. Matériau à libération percutanée selon la revendication 1,
dans lequel la teneur desdites particules de polymère dans la solution aqueuse est de 0,1 pour cent en poids à 50 pour cent en poids.

5. Une composition d'agent d'application externe comprenant le matériau à libération percutanée conforme à l'une des revendications 1 à 4.

6. Une composition d'agent d'application externe selon la revendication 5,
dans laquelle la teneur dudit matériau à libération percutanée est de 0,0001 pour cent en poids à 50 pour cent en poids.

7. Une composition d'agent d'application externe selon la revendication 5,
dans laquelle la formulation de la composition d'agent est un rafraîchisseur de peau, une préparation hydratante, une crème de massage, une crème nutritive, un enveloppement, un gel, un cosmétique d'adhérence à la peau, un rouge à lèvres, une base de maquillage, un fond de teint, un shampoing, un rinçage, un démaquillant corporel, un savon, une pâte dentifrice, un rince-bouche, des formulations pour la croissance capillaire, une lotion, un onguent, un gel, une crème, un patch ou un spray coiffant.

8. Un procédé pour préparer le matériau à libération percutanée de la revendication 1, comprenant les étapes de :
1) Préparation d'une phase huile en agitant le polymère ayant un poids moléculaire de 5 000 à 1 000 000 et un agent physiologiquement actif ;
2) Préparation d'une première émulsion en mélangeant ladite phase huile et de l'eau distillée dans laquelle est dissous un agent tensio-actif ;
3) Préparation d'une seconde émulsion en émulsifiant ladite première émulsion par un émulsifiant sous la condition que la pression est de 500 bar à 1 500 bar et la vitesse d'écoulement est de 20 ml/min à 150 ml/min ; et
4) Durcissement de ladite seconde émulsion.
